# EUROPEAN PATENT APPLICATION

(11) **EP 4 841 640 A1**
(43) Date of publication of application: **18.11.2026**
(21) Application number: 25176517.8
(22) Date of filing: 14.05.2025
(51) Int. Cl.: G16H 10/60, G16H 40/63

(54) **METHODS AND SYSTEMS TO IMPROVE CORRELATION BETWEEN SUBJECT SYMPTOMS AND ECG TRACING**

(71) Applicant: Koninklijke Philips N.V., 5656 AE Eindhoven (NL)
(72) Inventor: STUT, Wilhelmus Johannes Joseph, 5656 AG Eindhoven (NL); LACROIX, Joyca Petra Wilma, 5656 AG Eindhoven (NL); MILLER, Lacey Lynn Murdock, 5656 AG Eindhoven (NL)
(74) Representative: Philips Intellectual Property & Standards

(57) **Abstract**

A method (100) for recording symptoms experienced by a subject during ECG monitoring, comprising: receiving (120), by an app (280), a command to open or activate a symptom recording module (340) of the app; recording (130) a first time point comprising a time at which the command to open or activate the application was received; presenting (140) a start time entry screen prior to presenting any other screen requesting symptom information; receiving (150), from the start time entry screen, an entry of a start time for the symptoms experienced by the subject; generating (160), using the first time point and the start time, an adjusted start time; presenting (170) a symptom entry screen; receiving (180) from the symptom entry screen, an entry of one or more of the plurality of possible symptoms; and associating (190) the adjusted start time with the entered one or more of the plurality of possible symptoms.

## Description

### FIELD OF THE INVENTION

The disclosed subject matter generally pertains to methods and symptoms for recording symptoms experienced by a subject being monitored by ECG, and associating a more accurate start time with the symptoms.

### BACKGROUND OF THE INVENTION

Electrocardiogram (ECG) is a non-invasive test that measures the electrical activity of the heart by placing electrodes on the chest and limbs. It is a vital diagnostic tool in cardiology, used to identify a wide range of cardiac conditions, including heart attack, arrhythmia, and heart failure. ECG can also be used to monitor the effectiveness of treatment and detect early signs of complications.

Ambulatory ECG monitors collect ECG data outside the hospital setting (e.g., in the patient's home environment or on-the-go). Typically, an ECG sensor is applied to the patient's chest, and when they feel a symptom such as chest pain or shortness of breath, they can use a computer application ("app") to describe the symptoms and the activity they were engaged in when the symptoms occurred. The ECG data and symptom recordings are shared with the referring physician via an ECG report provided by the vendor.

It is vital for the referring physician or another entity interpreting the ECG data and symptom recordings that, in order for there to be good correlation between symptoms and ECG tracings, there be an accurate timestamp of the start of the symptom in the report. In other words, the timestamp in the report indicating the start of the symptoms should be as close as possible to the moment the patient actually started to feel the symptom.

However, for a variety of reasons, the timestamp indicating the start of the symptoms is not closely associated with the actual moment the patient actually started to feel the symptoms (the "start time "of the symptoms). For example, some apps used to record symptoms do not allow the user to enter the start time at all. Instead, the app registers the moment the patient recorded the symptom, which may be minutes, hours, or even days after the symptom started. Other apps that do allow the user to enter the start time only do so after many other steps of the process including selecting or entering the symptoms experienced, and/or the activity they were engaged in. Indeed, the patient needs time to become aware of and interpret their bodily signals (a process referred to as interoception), time to interact with the app to record symptoms and activities, and then time to finally record the start time. This significant time delay between experiencing symptoms and finally recording the start time strongly negatively affects the accuracy of the recording symptom start time and the correlation.

### SUMMARY OF THE INVENTION

Thus, there is a continued need to determine and associate a more accurate symptom start time with the actual onset for the one or more experienced symptoms. This results in significant improvements in analysis of the ECG trace for a subject.

Various embodiments and implementations are directed to methods and systems for recording symptoms experienced by a subject being monitored by ECG, and associating a more accurate start time with the symptoms. An app of an ECG monitoring system receives a command to open or activate its symptom recording module, and records a first time point comprising a time at which the command to open or activate the symptom recording module was received. The symptom recording module presents the subject with a start time entry screen, via which the subject enters a start time for the one or more symptoms. The system also receives information about the symptoms experienced by the subject, including one or more of an identity of the symptom(s) and the activity the subject was undergoing when the symptom occurred. The system associates in memory the recorded first time point with the received one or more symptoms.

According to an aspect, a method for recording one or more symptoms experienced by a subject during ECG monitoring is provided. The method includes: (i) receiving, by an app, a command to open or activate its symptom recording module; (ii) recording, by the symptom recording module, a first time point comprising a time at which the command to open or activate the symptom recording module was received; (iii) presenting to the subject a start time entry screen, wherein the start time entry screen is presented to the subject prior to presenting any other screen requesting symptom information; (iv) receiving, from the subject via the start time entry screen, an entry of a start time for the one or more symptoms experienced by the subject; (v) generating, using the first time point and the start time, an adjusted start time; (vi) presenting to the subject a symptom entry screen, wherein the symptom entry screen displays a plurality of possible symptoms; (vii) receiving, from the subject via the symptom entry screen, an entry of one or more of the plurality of possible symptoms; and (viii) associating, in memory by the symptom recording module, the adjusted start time with the entered one or more of the plurality of possible symptoms.

In accordance with an embodiment, the method further includes presenting to the subject a refining start time entry screen comprising a request for a more specific start time for the one or more symptoms experienced by the subject; and receiving, from the subject, an entry of a refined start time for the one or more symptoms experienced by the subject.

In accordance with an embodiment, the entry of the start time for the one or more symptoms experienced by the subject comprises a selection by the subject of a start time from among a plurality of possible start times.

In accordance with an embodiment, each of the plurality of possible start times comprises a different period of time from the current moment to a start time for the one or more symptoms experienced by the subject.

In accordance with an embodiment, the entry of the one or more symptoms experienced by the subject comprises a selection by the subject of one or more of the one or more of the plurality of possible symptoms.

In accordance with an embodiment, the entry of the start time for the one or more symptoms experienced by the subject comprises an entry of a specific start time for the one or more symptoms experienced by the subject.

In accordance with an embodiment, the method further includes displaying one or more of the adjusted start time and the entered one or more of the plurality of possible symptoms. In accordance with an embodiment, displaying further comprises an indication of a difference between one or more of the recorded first time point, the refined start time, and the adjusted start time.

According to an aspect, a system for recording one or more symptoms experienced by a subject during ECG monitoring is provided. The system includes: an ECG monitor worn by the subject; and an app, wherein the app is configured to: receive, from the subject wearing the ambulatory ECG monitor, a command to open or activate the symptom recording module; record a first time point comprising a time at which the command to open or activate its symptom recording module was received; present to the subject a start time entry screen, wherein the start time entry screen is presented to the subject prior to presenting any other screen requesting symptom information; receive, from the subject via the start time entry screen, an entry of a start time for the one or more symptoms experienced by the subject; present to the subject a symptom entry screen, wherein the symptom entry screen displays a plurality of possible symptoms; receive from the subject entry of one or more of the plurality of possible symptoms; and associate, in memory, the recorded first time point with the received entry of one or more of the plurality of possible symptoms.

In accordance with an embodiment, the app is further configured to: present to the subject a refining start time entry screen comprising a request for a more specific start time for the one or more symptoms experienced by the subject; and receive from the subject an entry of a refined start time for the one or more symptoms experienced by the subject.

In accordance with an embodiment, the system further includes a user interface configured to display one or more of the adjusted start time and the entered one or more of the plurality of possible symptoms. In accordance with an embodiment, displaying further comprises an indication of a difference between one or more of the recorded first time point, the refined start time, and the adjusted start time.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1A is a method for recording one or more symptoms experienced by a subject wearing being monitored by ECG, in accordance with an embodiment.
FIG. 1B is a method for recording one or more symptoms experienced by a subject wearing being monitored by ECG, in accordance with an embodiment.
FIG. 2 is a schematic representation of an ECG monitoring system, in accordance with an embodiment.
FIG. 3 is a schematic representation of an ECG monitoring environment, in accordance with an embodiment.
FIG. 4 is a schematic representation of a portion of a symptom recording module, in accordance with an embodiment.
FIG. 5 is a schematic representation of the order of the screens requesting symptom information of a symptom recording module, in accordance with an embodiment.
FIG. 6 is a schematic representation of an ECG trace, in accordance with an embodiment.
FIG. 7 is a schematic representation of a portion of a symptom recording module, in accordance with an embodiment.

### DETAILED DESCRIPTION OF EMBODIMENTS

The present disclosure describes various embodiments of a system and method configured for recording symptoms experienced by a subject being monitored by ECG, and associating a more accurate start time with the symptoms. More generally, Applicant has recognized and appreciated that it would be beneficial to provide methods and systems for improved ECG monitoring of a subject. An app of an ECG monitoring system receives a command to open or activate its symptom recording module, and records a first time point comprising a time at which the command to open or activate the symptom recording module was received. The system presents the subject with a start time entry screen, via which the subject enters a start time for the one or more symptoms. The system also receives information about the symptoms experienced by the subject, including one or more of an identity of the symptom(s) and the activity the subject was undergoing when the symptom occurred. The system associates in memory the recorded first time point with the received one or more symptoms.

According to an embodiment, the systems and methods described or otherwise envisioned herein can, in some non-limiting embodiments, be implemented as a component of or an extension of ECG systems. For example, one application of the embodiments and implementations herein is to improve systems such as, e.g., the Philips^{®} ECG and/or IntelliSpace^{®} line of diagnostic and reporting tools (manufactured by Koninklijke Philips, N.V.), among many other products. However, the disclosure is not limited to these devices or systems, and thus the disclosure and embodiments disclosed herein can encompass any ECG method, device, or system.

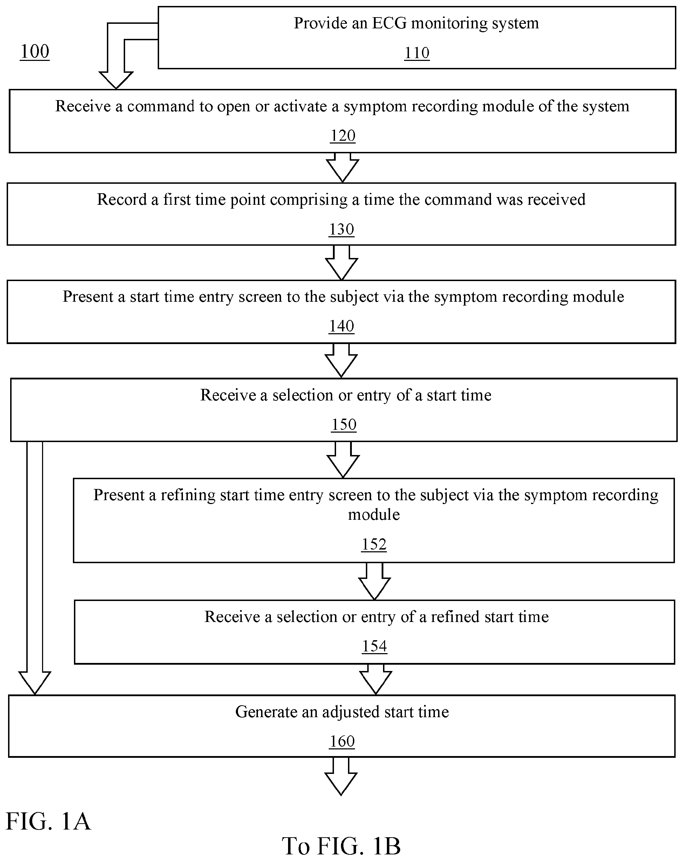

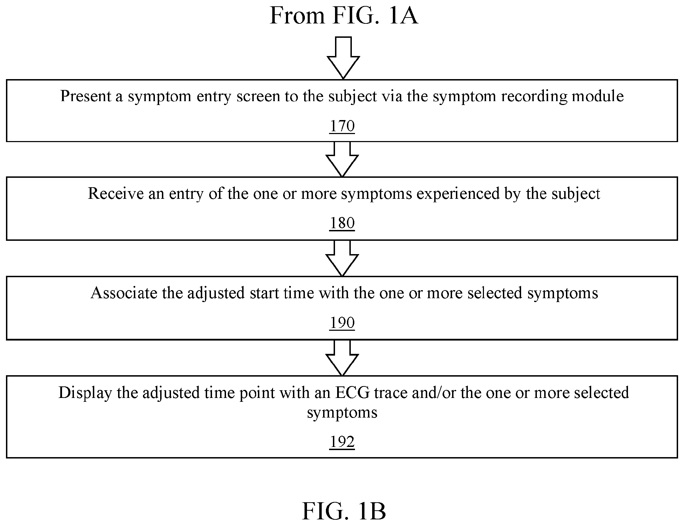

Referring to FIG. 1, in one embodiment, is a flowchart of a method 100 for recording one or more symptoms experienced by a subject during ECG monitoring using an ECG monitoring system. The methods described in connection with the figures are provided as examples only, and shall be understood to not limit the scope of the disclosure. The ECG monitoring system can be any of the systems described or otherwise envisioned herein. The ECG monitoring system can be a single system or multiple different systems.

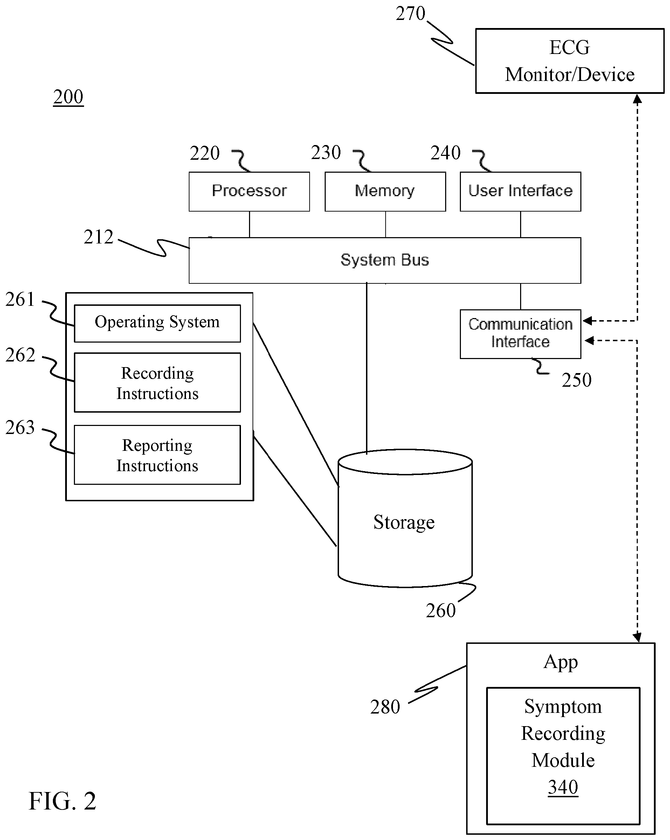

At step 110 of the method, an ECG monitoring system 200 is provided. Referring to an embodiment of an ECG monitoring system 200 as depicted in FIG. 2, for example, the system comprises one or more of a processor 220, memory 230, user interface 240, communication interface 250, and storage 260, interconnected via one or more system buses 212. It will be understood that FIG. 2 constitutes, in some respects, an abstraction and that the actual organization of the components of the system 200 may be different and more complex than illustrated. Additionally, ECG monitoring system 200 can be any of the systems described or otherwise envisioned herein. Other elements and components of ECG monitoring system 200 are disclosed and/or envisioned elsewhere herein.

According to an embodiment, the ECG monitoring system 200 is or comprises or is in local and/or remote communication with an ECG monitor or device 270. The ECG monitor or device 270 can be any ECG monitor or device, including but not limited to an ambulatory ECG monitor or device. An ambulatory ECG monitor or device is typically worn by a patient or subject for a period of time (hours, days, or weeks) as the patient or subject goes about their daily life, typically at home and work. Once the monitoring time period is complete, the patient or subject can provide the recorded ECG information to a healthcare professional. For example, the ECG monitor or device can upload the recorded ECG information (in real-time or in batches or all at once), or the patient or subject can return the ambulatory ECG monitor or device to the healthcare professional for download of the recorded ECG information. Notably, however, the methods and systems described or otherwise envisioned herein are not limited to ambulatory ECG monitors or devices.

According to an embodiment, the ECG monitor or device 270-such as a Holter monitor or a patch-based monitor-continuously captures the electrical activity of the subject's heart over an extended period, often 24 hours or more. While the subject wears the monitor, which is typically lightweight and designed for everyday activities, the subject also has access to an application - such as a smartphone app - that plays a crucial role in the cardiac evaluation. The app allows the subject to record details about their symptoms and activities throughout the day, providing essential context to the ECG data.

For example, whenever the subject experiences an unusual symptom, such as palpitations (a sudden sensation of a rapid or irregular heartbeat), chest pain or discomfort, dizziness, shortness of breath, or even episodes of sweating or anxiety, the subject can open the app and mark the event in real time. The app typically includes fields where the subject can add notes about the intensity and nature of the symptom. The healthcare provider will later attempt to correlate these reports with the ECG recordings to identify any patterns or triggers associated with the symptoms.

**In** addition to symptoms, the subject can also log various activities that might influence their heart rate. For example, the subject might record periods of exercise, such as walking, running, or climbing stairs; note when they are resting or sleeping; or describe stressful situations, like dealing with heavy traffic or tackling a challenging task at work. The subject may also record their medication intake and/or dietary intake, such as consuming caffeine, alcohol, or a heavy meal, if they suspect these factors might trigger their symptoms. This detailed logging of both symptoms and activities is essential because it helps the healthcare provider better understand the context in which any abnormal heart rhythms occur.

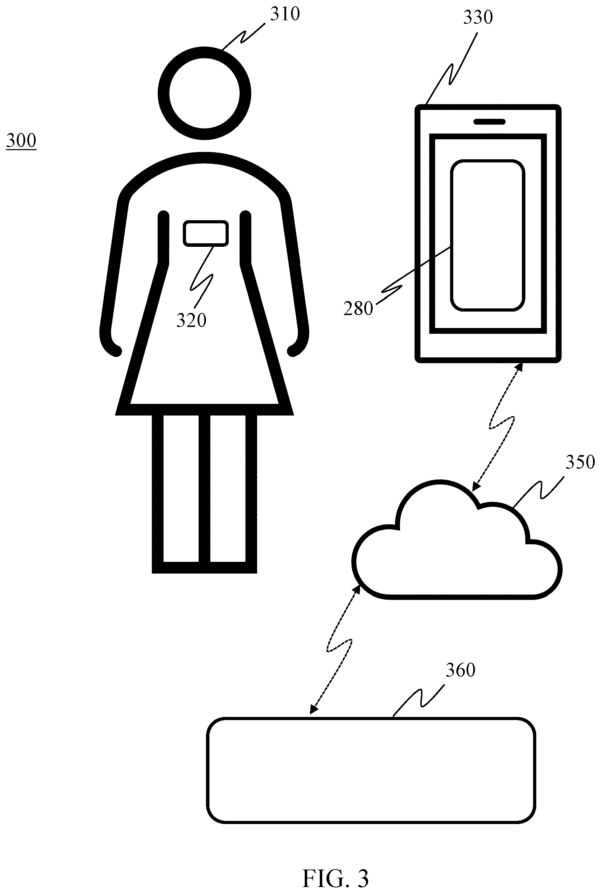

Referring to FIG. 3, in one embodiment, is an ECG monitoring environment 300. The ECG monitoring environment comprises a subject 310 that is being monitored using an ECG device 320, which may be an ambulatory ECG device. The environment further comprises a computerized device 330 with a software application ("app") 280, although the app may be hosted on any computerized device including but not limited to a smartphone, desktop, laptop, tablet, wearable, smart speaker, smart mirror, and many other possible devices.

According to an embodiment, the subject 310 feels a symptom - such as palpitation, chest pain or discomfort, dizziness, shortness of breath, sweating, or anxiety - and desires to record that symptom and the activity the subject was engaged in when the symptom started, in the app 280 with symptom recording module 340. Accordingly, the subject activates or triggers or opens the symptom recording module 340 and records one or more of the time the symptoms started, the symptoms (of which there may be one or more), and the activities that the subject was engaged in (of which there may be one or more). The app stores this information. The computerized device 330 and/or app 280 can transmit the stored information via a wired and/or wireless communication system 350 to a remote component 360 of the ECG monitoring system, such as a database or another application. This transmission of information can be in real-time, in batches, and/or all at once such as when monitoring is complete or the subject or healthcare professional directs or requests the transmission of the information.

Returning to method 100 in FIG. 1, at step 120 of the method, the app 280 receives a command to open or activate the app. According to an embodiment, the command is received from the subject being monitored, although a helper, family member, or other person may provide the command on behalf of the subject being monitored. For example, the subject may experience a symptom and record that symptom using the app. As one non-limiting example, the subject may experience palpitation while going for a run. Since the subject has been instructed, or otherwise knows, to record symptoms using the app, the subject uses their smartphone, wearable (such as a smart watch), or other device or component hosting the app to open or activate the app. As another non-limiting example, the subject may experience dizziness while walking up stairs and ask their spouse to record the symptom and activity using the app. Many other configurations and situations are possible.

According to an embodiment, the command to open or activate the app is any command sufficient to open or activate an app. For example, the subject or other person may tap, click, select, voice-activate, or otherwise activate an app on the computerized device or other component hosting the app to open or activate the app. As one non-limiting example, the subject may pull their smartphone from their pocket, find the app, and click it to open it.

At step 130 of the method, the symptom recording module 340 automatically records a first time point, comprising the time at which symptom recording module was opened or activated. For example, the user may experience the symptom and activate or open the app by clicking on the app at 11:23:47 AM. The symptom recording module will record this time, and will correlate that time with the symptoms and activities provided or selected by the subject, as described or otherwise envisioned herein. To record a first time point, the symptom recording module can save the time point in a data storage structure, along with the symptoms and activities provided or selected by the subject. Although the app 280 with symptom recording module 340 is a unique, novel, and non-generic app that is programmed or designed to perform the novel and non-obvious methods described or otherwise envisioned herein, the data storage structure can be any structure that stores data such as a time point, symptom, and/or activity.

Recording a first time point comprising the time at which the symptom recording module was opened or activated provides a vital improvement of the ECG monitoring system 200 over existing ECG monitoring systems. Since the ECG monitoring system records the time at which the module is opened or activated, this is a significant improvement over systems that do not record the activation time as described or otherwise envisioned herein. It is vital, for accurate interpretation of ECG results, to correlate ECG traces with symptoms experienced by the person being monitored (and their activities), which requires an accurate record of the time during which the symptoms were experienced. If there is an incomplete or inaccurate correlation, meaning that the time is incorrect or inaccurate, then it will be significantly harder - and sometimes impossible - for the clinician or healthcare provider to review the ECG trace accurately.

According to an embodiment, the first time point is the current date-time retrieved via a *Date* function, and is recorded as a variable such as t_{startSymptomRecording}, although other variable names are possible.

At step 140 of the method, the subject is presented with a start time entry screen. According to an embodiment, the start time entry screen is presented to the subject via a user interface of the system. For example, most applications comprise a plurality of displays, screens, or other presentation of information, through either static or dynamic content. In one embodiment, the start time entry screen includes one or more interactive elements - such as a slider, button, dropdown, clock interface, or other element - that allows the selection or entry of a start time for the one or more symptoms. According to an embodiment, the start time entry screen is any screen or interface that allows the subject to select or enter the start time for the one or more symptoms (i.e., the moment the subjects started feeling the one or more symptoms).

According to an embodiment, the start time entry screen is presented to the subject prior to presenting any other screen requesting symptom information. This again provides a vital improvement of the ECG monitoring system 200 over existing ECG monitoring systems, as it more closely correlates or associates the time the one or more symptoms started, with those symptoms and the activities in which the subject was engaged.

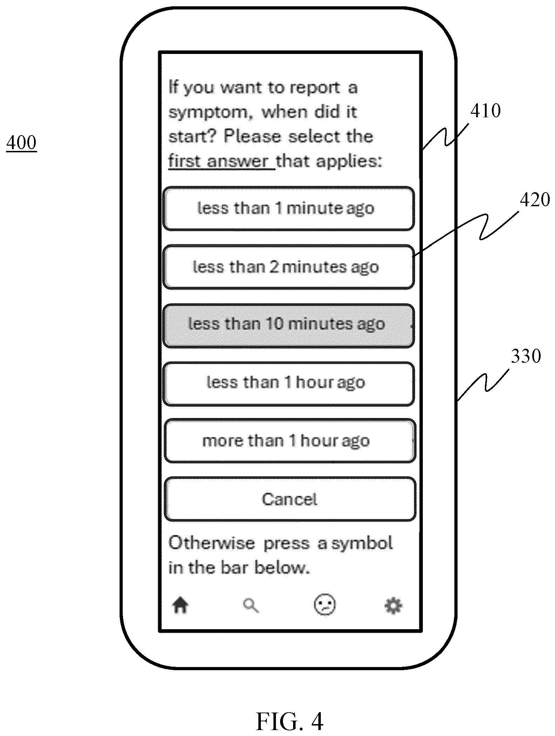

As just one non-limiting example, FIG. 4 shows a portion 400 of an ECG monitoring system in which start time entry screen 410 is presented to the subject by the symptom recording module 340. The start time entry screen 410 is displayed on the user interface - such as the display or screen - of a computerized device 330 which can be a smartphone or any other computerized device as described or otherwise envisioned herein. The start time entry screen 410 can comprise a prompt (e.g., "If you want to report a symptom, when did it start? Please select the first answer that applies:"). The start time entry screen 410 can also comprise one or more interactive elements 420 that allow the selection or entry of a start time for the one or more symptoms. In this non-limiting example, the interactive elements 420 comprise a plurality of time selection options, including: (1) less than 1 minute ago; (2) less than 2 minutes ago; (3) less than 10 minutes ago; (4) less than 1 hour ago; (5) more than 1 hour ago; and (6) Cancel. Of course, the time selection options can include more or different times than the ones provided, including (1) less than 1 minute ago; (2) 1-2 minutes ago; (3) 3-10 minutes ago; (4) 11-60 minutes ago; (5) more than 1 hour ago; and (6) Cancel. According to an embodiment, some or all of the screens or displays in the symptom recording module 340 has a cancel button or option in the event the subject wants to cancel the process or decides there was no symptom or does not want to report the symptom.

According to one possible embodiment, after presenting a start time entry screen to the subject via a user interface of the system, the selectable options/buttons can be automatically removed or disabled (and greyed out) after a corresponding delay if the subject has not made a selection yet. For example, the button "button "less than 1 minute ago" can be automatically removed or disabled 1 minute after opening the start time entry screen (=t_{startSymptomRecording}), the button "less than 2 minutes ago" can be automatically removed or disabled 2 minutes after opening the start time entry screen (=t_{startSymptomRecording}), and so on.

At step 150 of the method, in accordance with an embodiment, the symptom recording module receives an entry of a start time for the one or more symptoms experienced by the subject. According to an embodiment, the module receives the entry from the subject or another user via the user interface of the computerized device and/or app. For example, the subject may click on or otherwise select an interactive element on the start time entry screen 410. Other methods or processes for receiving or providing the start time for the one or more symptoms experienced by the subject are possible.

At optional step 152 of the method, the subject is presented with a refining start time entry screen comprising a request for a more specific start time for the one or more symptoms experienced by the subject. According to an embodiment, the start time entry screen is presented to the subject via a user interface of the system. According to an embodiment, the refining start time entry screen is any screen or interface that allows the subject to select or enter the refined start time for the one or more symptoms. For example, the refining start time entry screen a dynamic or interactive time picker.

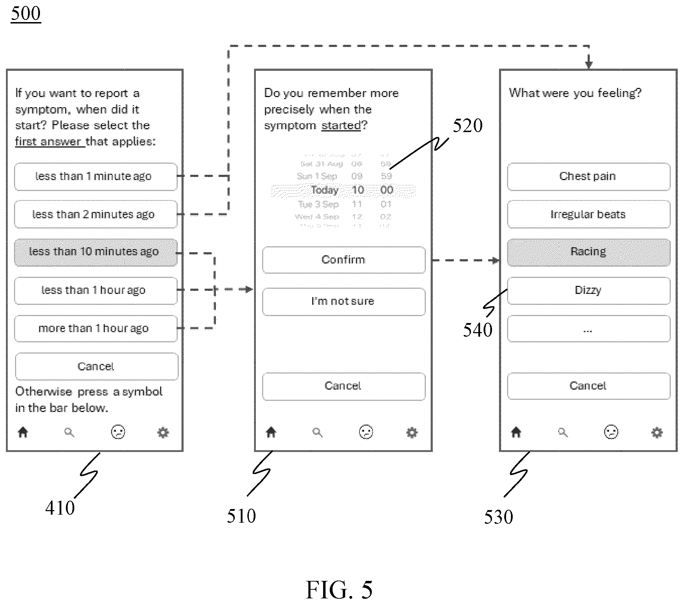

As just one non-limiting example, FIG. 5, shows a portion 500 of an ECG monitoring system in which refining start time entry screen 510 is presented to the subject by the symptom recording module 340. The refining start time entry screen 510 is displayed on the user interface - such as the display or screen - of a computerized device 330 which can be a smartphone or any other computerized device as described or otherwise envisioned herein. The start time entry screen 410 can comprise a prompt (e.g., "Do you remember more precisely when the symptom started?"). The refining start time entry screen 510 can also comprise one or more interactive elements 520 that allow the selection or entry of a refined start time for the one or more symptoms. In this non-limiting example, the interactive elements 520 comprise a time picker by which the subject is presented with a default time (which can be the current time or a predetermined default time, among other options) and the subject slides or otherwise manipulates the time picker to arrive at a refined start time. However, other interactive elements are possible. Indeed, according to one embodiment, the subject can enter a time by voice or thought to control the time picker or otherwise provide the time.

In this non-limiting embodiment, the subject is presented with the refining start time entry screen based on the start time selected or provided via the start time entry screen (such as by selection of an interactive element), where some selected start times do not trigger use or presentation of the refining start time entry screen, and other selected start times do trigger use or presentation of the refining start time entry screen. However, according to other embodiments, the subject may be presented with the refining start time entry screen every time or never, among other variations.

Thus, for example, in the non-limiting embodiment shown in FIG. 5, the subject is presented with the refining start time entry screen 510 only if the subject selects or enters certain start times on the start time entry screen 410, such as "less than 10 minutes ago," "less than 1 hour ago," and "more than one hour ago." Other selections such as "less than 1 minute ago" and "less than 2 minutes ago" bypass the refining start time entry screen 510.

Per the non-limiting embodiment shown in FIG. 5, the time picker is shown with the time at which the symptom recording module was opened or activated (in this example 10:00), although the subject may adjust the default time or it may be the current time, among other options.

At optional step 156 of the method, in accordance with an embodiment, the symptom recording module receives an entry of a refining start time for the one or more symptoms experienced by the subject. According to an embodiment, the module receives the entry from the subject or another user via the user interface of the computerized device and/or app. For example, the subject may click on or otherwise select an interactive element on the refining start time entry screen 510, such as scrolling to select a time using the time picker. Other methods or processes for receiving or providing the refined start time for the one or more symptoms experienced by the subject are possible.

According to the non-limiting embodiment in FIG. 5, the subject presses or selects "Confirm" to enter the highlighted time as the refining start time for the one or more symptoms, and that refined time is stored by the app in memory as variable tₜᵢₘₑₛₜₐₘₚ, although other variable names are possible.

At step 160 of the method, the system - such as the symptom recording module - generates an adjusted start time for the one or more symptoms experienced by the subject using the recorded first time point and the received start time entered via the start time entry screen, or optionally uses the refined start time entered via the refining start time entry screen. This can be accomplished in a variety of different ways, including but not limited to subtracting the recorded first time point from an amount of time (such as the selected start time, e.g., "less than 1 minute ago"). Many other methods are possible.

According to an embodiment, the system configures a time picker with a default time to minimize the effort and time needed for the subject to report the time the one or more symptoms started. Depending on the answer selected by the subject, the symptom recording module 340 responds accordingly, with a variable such as tₜᵢₘₑₛₜₐₘₚ (other labels are possible) representing the timestamp of the symptom in the database and *round* being a standard function yielding the nearest minute (e.g., *round*(10:04:21) *=* 10:04). TABLES 1 and 2 show one possible embodiment of this response.

**TABLE 1. Response of the symptom recording module.**

| Subject-Selected Answer | Application Response |
|---|---|
| less than 1 minute ago | tₜᵢₘₑₛₜₐₘₚ :=t_{startSymptomRecording} |
| less than 2 minutes ago | tₜᵢₘₑₛₜₐₘₚ :=t_{startSymptomRecording} - 1 minute |
| less than 10 minutes ago | show time picker with default time *round*(t_{startSymptomRecording}) - m₁ minutes |
| less than 1 hour ago | show time picker with default time *round*(t_{startSymptomRecording}) - m₂ minutes |
| more than 1 hour ago | show time picker with default time *round*(t_{startSymptomRecording}) - m₃ minutes |

For example, if the values 4, 25, and 90 are chosen for m₁, m₂, and m₃, respectively, and the patient opens the app at 10:04:21, the app behaves as follows. Note that *round*(t_{startSymptomRecording}) = *round*(10:04:21) *=* 10:04.

**TABLE 2. Response of the symptom recording module.**

| Subject-Selected Answer | Application Response |
|---|---|
| less than 1 minute ago | tₜᵢₘₑₛₜₐₘₚ = 10:04:21 |
| less than 2 minutes ago | tₜᵢₘₑₛₜₐₘₚ = 10:03:21 |
| less than 10 minutes ago | show time picker with default time 10:00 (= 10:04 - 4 minutes) |
| less than 1 hour ago | show time picker with default time 09:39 (= 10:04 - 25 minutes) |
| more than 1 hour ago | show time picker with default time 08:34 (= 10:04 - 90 minutes) |

The above responses are only provided as non-limiting examples. Instead of showing five possible interactive elements on the time entry screen, the app may show more or fewer, or different, interactive elements. Additionally, the TABLE 1 and 2 the number of minutes that are subtracted from *round*(t_{startSymptomRecording}) when the subject selects *less than 10 minutes ago* or *less than 1 hour ago* have been chosen in such a way that the time picker initially shows the time that is halfway the selected answer and the previous answer. For example, when the subject selects *less than 10 minutes ago*, (10 - 2) / 2 = 4 minutes are subtracted. By initially showing the "halfway time" the effort to select the start moment of the symptom is further reduced (less scrolling needed on average). A different number of minutes may be subtracted from t_{startSymptomRecording} (e.g., 5 minutes when the patient selects *less than 10 minutes ago*)*.* Other variations are possible.

According to an embodiment, if the subject selects "*I'm not sure*" the symptom may be associated with two timestamps in the database: (1) t_{timestamp_1} :=*round*(t_{startSymptomRecording}) - the number of minutes mentioned in the *selected* answer; and (2) t_{timestamp_2} :=*round*(t_{startSymptomRecording}) - the number of minutes mentioned in the *preceding* answer. Thus, for example, if the patient selects *less than 10 minutes ago* and then "*I'm not sure*", t_{timestamp_1} gets value 09:54 (= 10:04 - 10 minutes) and t_{timestamp_2} gets value 10:02 (= 10:04 - 2 minutes) indicating that the symptom started between 09:54 (10 minutes ago) and 10:02 (2 minutes ago).

According to yet another embodiment, an alternative formula could be used if the subject selects *less than 2 minutes ago* (or *1-2 minutes ago*, which is the same). See, for example, TABLE 3.

**TABLE 3. Alternative response of the symptom recording module.**

| Subject-Selected Answer | Application Response |
|---|---|
| less than 1 minute ago | tₜᵢₘₑₛₜₐₘₚ :=t_{startSymptomRecording} |
| less than 2 minutes ago (or 1-2 minutes ago) | tₜᵢₘₑₛₜₐₘₚ :=minimum ( t_{startSymptomRecording}, the moment the subject selected this answer - 60s) |
| ... | ... |

At step 170 of the method, the subject is presented with a symptom entry screen that displays a plurality of possible symptoms. According to an embodiment, the symptom entry screen is presented to the subject via a user interface of the system. For example, most applications comprise a plurality of displays, screens, or other presentation of information, through either static or dynamic content. In one embodiment, the symptom entry screen includes one or more interactive elements - such as buttons, a dropdown, or other element - that allows the selection or entry of one or more symptoms experienced by the subject. According to an embodiment, the symptom entry screen is any screen or interface that allows the subject to select or enter one or more symptoms.

As just one non-limiting example, FIG. 5 shows a portion 400 of an ECG monitoring system in which symptom entry screen 530 is presented to the subject by the symptom recording module 340. The symptom entry screen 530 is displayed on the user interface - such as the display or screen - of a computerized device 330 which can be a smartphone or any other computerized device as described or otherwise envisioned herein. The start time entry screen 410 can comprise a prompt (e.g., "What were you feeling?"). The symptom entry screen 530 can also comprise one or more interactive elements 540 that allow the selection or entry of one or more symptoms. In this non-limiting example, the interactive elements 540 comprise a plurality of symptom selection options, including: (1) Chest pain; (2) Irregular beats; (3) Racing; and (4) Dizzy. Of course, the symptom selection options can include more or different symptoms than the ones provided. The subject can select one symptom or multiple symptoms via the symptom entry screen.

At step 180 of the method, in accordance with an embodiment, the symptom recording module receives, via the symptom entry screen, an entry of the one or more symptoms experienced by the subject. According to an embodiment, the app receives the entry from the subject or another user via the user interface of the computerized device and/or app. For example, the subject may click on or otherwise select an interactive element on the symptom entry screen 530. Other methods or processes for receiving or providing the one or more symptoms experienced by the subject are possible.

At optional step 190 of the method, in accordance with an embodiment, the symptom recording module and/or the computerized device associate the adjusted start time with the one or more symptoms selected or entered by the subject using the symptom entry screen. This association can be done in memory using any method for associating two or more data points. For example, the association can be done in a data storage structure of the computerized device.

At optional step 192 of the method, the system may display, such as on an ECG user interface or any other user interface, the adjusted time point together with the ECG trace and/or the one or more symptoms experienced by the subject. According to an embodiment, the display may include an indication of a difference between the adjusted time point and one or more of the first time point and the refined time point. The provided output can be any of the information as described or otherwise envisioned herein. The system may provide the information to a user via any mechanism, including but not limited to a visual display. The information may be communicated by wired and/or wireless communication to another device. For example, the system may communicate the information to a monitor, screen, mobile phone, computer, laptop, wearable device, and/or any other device configured to allow display and/or other communication of the information.

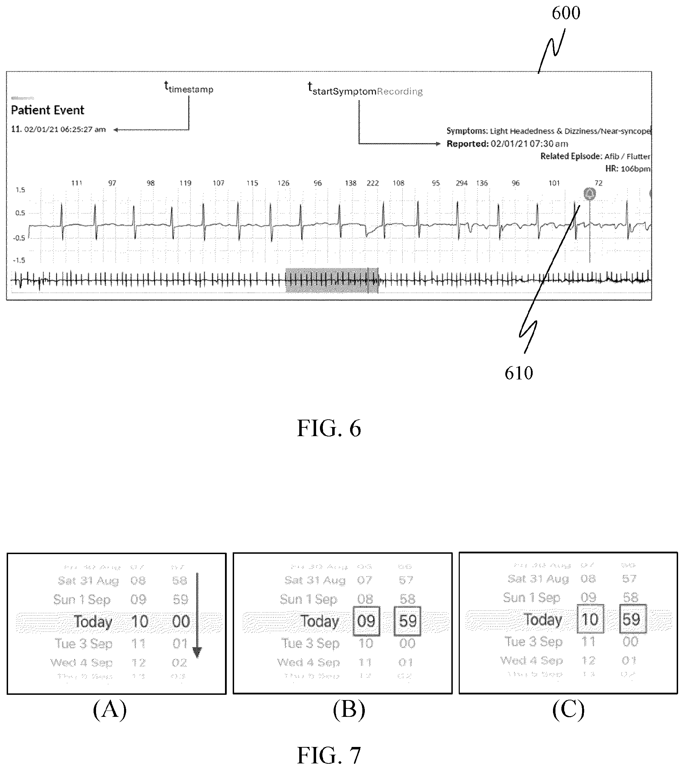

According to an embodiment, when symptoms are recorded retrospectively, the time selected by the subject may be less accurate, especially when this time is further in the past. To deal with this, the screen with the time picker can optionally offer the option to select "I'm not sure." Still, subjects may not press this button as they know the exact time they started feeling the symptom, for example, because they looked at their watch and wrote down this time. Or they think they know the exact time they started feeling the symptom. When recording a symptom retrospectively, the app does not know the accuracy of the time selected by the patient. To deal with potential inaccuracy, the symptom in the database not only has attribute tₜᵢₘₑₛₜₐₘₚ but also attribute t_{startSymptomRecording}. The report may show both values, as shown in FIG. 6 which is an ECG trace displayed on a display, comprising both tₜᵢₘₑₛₜₐₘₚ and t_{startSymptomRecording}.

According to an embodiment, depending on the difference between t_{startSymptomRecording} and tₜᵢₘₑₛₜₐₘₚ, the report may show tₜᵢₘₑₛₜₐₘₚ and/or t_{startSymptomRecording} so that the physician is informed about this difference. For example, tₜᵢₘₑₛₜₐₘₚ may have a textual or color annotation according to the TABLE 4 (although other textual or color annotations are possible). Alternatively, the circle with the bell (610 in FIG. 6) which denotes the symptom, can have a different color according to the TABLE 4 (although other textual or color annotations are possible).

**TABLE 4. Display Annotations**

| Difference between the moment the symptom recording started and the timestamp selected by the subject | Textual annotation | Color annotation |
|---|---|---|
| t_{startSymptomRecording} - tₜᵢₘₑₛₜₐₘₚ ≤ 2 minutes | immediate | GREEN |
| 2 mins < t_{startSymptomRecording} - tₜᵢₘₑₛₜₐₘₚ ≤ 15 mins | promptly | BLUE |
| 15 mins < t_{startSymptomRecording} - tₜᵢₘₑₛₜₐₘₚ | delayed | RED |

According to an embodiment, a patient may be recommended (by a care provider, a manual, and/or the app) to open the app as soon as they feel something that could be a symptom; they can always cancel the symptom recording if they conclude the feeling is not a symptom and decide not to record it. However, some patients may interpret this recommendation as an encouragement to record every small feeling, which may lead to an overload of clinically irrelevant symptoms. Hence, this recommendation is only given if the care provider estimates that a patient will interpret the recommendation wisely.

According to an embodiment, to reduce the time needed to select the start moment of the symptom, the time picker can scroll through the hour, such as shown in FIG. 7. For example, when the time picker highlights 10:00 and the patient moves the minute wheel to 59 (see FIG. 7, panel A), the time picker should show 09:59 (see FIG. 7, panel B), instead of 10:59 (see FIG. 7, panel C). Many other variations are possible.

Referring again to FIG. 2 is a schematic representation of an ECG monitoring system 200. System 200 may be any of the devices or systems described or otherwise envisioned herein, and may comprise any of the components described or otherwise envisioned herein. It will be understood that FIG. 2 constitutes, in some respects, an abstraction and that the actual organization of the components of system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, storage 260 of system 200 may store one or more algorithms, modules, and/or instructions to carry out one or more functions or steps of the methods described or otherwise envisioned herein. For example, storage 260 may comprise, among other instructions or data, recording instructions 262 and reporting instructions 263.

According to an embodiment, recording instructions 262 direct the system to receive or obtain and record a first time point comprising a time at which the command to open or activate the symptom recording module was received, receive or obtain and record an entry of a start time for the one or more symptoms experienced by the subject, receive or obtain and record an entry of one or more symptoms, and associate an adjusted start time with the one or more symptoms, among other possible variations and steps, instructions, information, or commands.

According to an embodiment, reporting instructions 263 direct the system to provide an output- such as an ECG trace and information about symptoms, activities, and/or timing - to a patient, clinician, or to another device or system. The provided output can be any of the information as described or otherwise envisioned herein. The system may provide the information to a user via any mechanism, including but not limited to a visual display. The information may be communicated by wired and/or wireless communication to another device. For example, the system may communicate the information to a monitor, screen, mobile phone, computer, laptop, wearable device, and/or any other device configured to allow display and/or other communication of the information.

It should be appreciated that all combinations of the foregoing concepts and additional concepts discussed in greater detail herein (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. **In** the latter scenario, the remote computer can be connected to the user's computer through any type of network, comprising a local area network (LAN) or a wide area network (WAN), or the connection can be made to an external computer (for example, through the Internet using an Internet Service Provider). **In** some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for recording one or more symptoms experienced by a subject during ECG monitoring, comprising:
receiving (120), by an app (280), a command to open or activate a symptom recording module (340) of the app;
recording (130), by the symptom recording module, a first time point comprising a time at which the command to open or activate the symptom recording module was received;
presenting (140) to the subject a start time entry screen, wherein the start time entry screen is presented to the subject prior to presenting any other screen requesting symptom information;
receiving (150) from the subject via the start time entry screen, an entry of a start time for the one or more symptoms experienced by the subject;
generating (160), using the first time point and the start time, an adjusted start time;
presenting (170) to the subject a symptom entry screen, wherein the symptom entry screen displays a plurality of possible symptoms;
receiving (180) from the subject via the symptom entry screen, an entry of one or more of the plurality of possible symptoms; and
associating (190), in memory by the symptom recording module, the adjusted start time with the entered one or more of the plurality of possible symptoms.

2. The method of claim 1, further comprising:
presenting (152) to the subject a refining start time entry screen comprising a request for a more specific start time for the one or more symptoms experienced by the subject; and
receiving (154) from the subject via the refining start time entry screen, an entry of a refined start time for the one or more symptoms experienced by the subject.

3. The method of claim 1, wherein the entry of the start time for the one or more symptoms experienced by the subject comprises a selection by the subject of a start time from among a plurality of possible start times.

4. The method of claim 3, wherein each of the plurality of possible start times comprises a different period of time from the current moment to a start time for the one or more symptoms experienced by the subject.

5. The method of claim 1, wherein the entry of the one or more symptoms experienced by the subject comprises a selection by the subject of one or more of the one or more of the plurality of possible symptoms.

6. The method of claim 2, wherein the entry of the start time for the one or more symptoms experienced by the subject comprises an entry of a specific start time for the one or more symptoms experienced by the subject.

7. The method of claim 2, further comprising:
displaying (192) one or more of the adjusted start time and the entered one or more of the plurality of possible symptoms.

8. The method of claim 7, wherein displaying further comprises an indication of a difference between one or more of the recorded first time point, the refined start time, and the adjusted start time.

9. A system (200) for recording one or more symptoms experienced by a subject during ECG monitoring, comprising:
an ECG monitor or device (270) worn by the subject; and
an app (280), wherein the app comprises a symptom recording module (340) and is configured to:
receive, from the subject wearing the ambulatory ECG monitor, a command to open or activate the symptom recording module;
record a first time point comprising a time at which the command to open or activate the symptom recording module was received;
present to the subject a start time entry screen, wherein the start time entry screen is presented to the subject prior to presenting any other screen requesting symptom information;
receive from the subject via the start time entry screen an entry of a start time for the one or more symptoms experienced by the subject;
present a symptom entry screen, wherein the symptom entry screen displays a plurality of possible symptoms;
receive from the subject via the symptom entry screen, an entry of one or more of the plurality of possible symptoms; and
associate, in memory, the recorded first time point with the received entry of one or more of the plurality of possible symptoms.

10. The system of claim 9, wherein the app is further configured to:
present to the subject a refining start time entry screen comprising a request for a more specific start time for the one or more symptoms experienced by the subject; and
receive from the subject via the refining start time entry screen, an entry of a refined start time for the one or more symptoms experienced by the subject.

11. The system of claim 9, wherein the entry of the start time for the one or more symptoms experienced by the subject comprises a selection by the subject of a start time from among a plurality of possible start times.

12. The symptom of claim 11, wherein each of the plurality of possible start times comprises a different period of time from the current moment to a start time for the one or more symptoms experienced by the subject.

13. The system of claim 9, wherein the entry of the one or more symptoms experienced by the subject comprises a selection by the subject of one or more of the one or more of the plurality of possible symptoms.

14. The system of claim 10, wherein the entry of the start time for the one or more symptoms experienced by the subject comprises an entry of a specific start time for the one or more symptoms experienced by the subject.

15. The system of claim 9, further comprising:
a user interface configured to display one or more of the adjusted start time and the entered one or more of the plurality of possible symptoms.
